# EUROPEAN PATENT SPECIFICATION

(11) **EP 2 705 159 B1**
(45) Date of publication and mention of the grant of the patent: **21.03.2018**
(21) Application number: 12724923.3
(22) Date of filing: 04.05.2012
(51) Int. Cl.: C12Q 1/68

(54) **METHODS FOR SEQUENCING, AMPLIFICATION AND DETECTION OF NUCLEIC ACIDS COMPRISING INTERNALLY LABELLED PRIMER**
VERFAHREN ZUR SEQUENZIERUNG, AMPLIFIKATION UND NACHWEIS VON NUKLEINSÄUREN MIT INTERN MARKIERTEM PRIMER
PROCÉDÉS DE SÉQUENÇAGE, D'AMPLIFICATION ET DE DÉTECTION D'ACIDES NUCLÉIQUES COMPRENANT UNE AMORCE MARQUÉE EN INTERNE

(30) Priority: 06.05.2011 EP 11165142
(43) Date of publication of application: 12.03.2014
(73) Proprietor: Qiagen GmbH, 40724 Hilden (DE)
(72) Inventor: DI PASQUALE, Francesca, 40724 Hilden (DE); MÜLLER, Daniel, 40724 Hilden (DE)
(74) Representative: CH Kilger Anwaltspartnerschaft mbB
(86) International application number: PCT/EP2012/058259
(87) International publication number: WO 2012/152698

(56) References cited:
- WO-A2-00/79009
- WO-A2-02/057479
- WO-A2-2006/053259
- PINCAS H ET AL: "High sensitivity EndoV mutation scanning through real-time ligase proofreading", NUCLEIC ACIDS RESEARCH, OXFORD UNIVERSITY PRESS, SURREY, GB, vol. 32, no. 19, 1 January 2004 (2004-01-01), page E148, XP002356609, ISSN: 0305-1048, DOI: 10.1093/NAR/GNH150
- SCHÜTZ E ET AL: "Genotyping of Eight Thiopurine Methyltransferase Mutations: Three-Color Multiplexing, Two-Color/Shared Anchor, and Fluorescence-quenching Hybridization Probe Assays Based on Thermodynamic Nearest-Neighbor Probe Design", CLINICAL CHEMISTRY, AMERICAN ASSOCIATION FOR CLINICAL CHEMISTRY, WASHINGTON, DC, vol. 46, no. 11, 1 January 2000 (2000-01-01), pages 1728-1737, XP003010931, ISSN: 0009-9147
- ECKERT C ET AL: "Potential of LightCycler technology for quantification of minimal residual disease in childhood acute lymphoblastic leukemia.", LEUKEMIA : OFFICIAL JOURNAL OF THE LEUKEMIA SOCIETY OF AMERICA, LEUKEMIA RESEARCH FUND, U.K FEB 2000 LNKD- PUBMED:10673751, vol. 14, no. 2, February 2000 (2000-02), pages 316-323, XP002661240, ISSN: 0887-6924

## Description

### Field of the Invention

The present invention is in the field of biology and chemistry, particularly in the field of molecular biology. More in particular, the invention relates to methods for the sequencing, or amplification and detection of nucleic acids.

### Background of the Invention

Today molecular methods play an important role in diagnostics and in identifying individuals, e.g. in the field of forensic sciences or in paternity testing. Amplification of target regions is a central procedure within these methods. Furthermore, the methods comprise an analysis of the length of amplified target regions, mostly by gel electrophoresis like capillary electrophoresis. The analysis of amplified material is performed via the detection of fluorescent labels. The labels are attached to the 5'-end of the site specific primers which are used for amplification. However, when analyzing the amplified products the appearance of small, impeding, artificial peaks, so called "dye blobs", results in a base line with a high "background noise" and this interferes with the sensitivity of the method. "Dye blobs" are peaks in the electropherograms that do not represent the specific amplification products, but degraded products comprising the label. The molecules causing these are free fluorescence dye labels as well as 1-8 bp long oligonucleotides linked to the label. The latter may represent degraded primers or degraded PCR-products.

PCT application WO 02/057479 discloses the use of internally labelled primers in sequencing and amplification reactions. The inventors now unexpectedly found that the use of internally labelled oligonucleotide primers diminishes the appearance of dye blobs. The present application, therefore, provides a method for sequencing, or amplifying and detecting nucleic acids comprising the use of internally labelled nucleic acid primers. The technical effect of the use of the internally labelled primers is the provision of a method with a higher sensitivity.

### Description of the Invention

Hence, the present invention solves the above-mentioned problems and provides for a method for sequencing, or amplifying and detecting nucleic acids consisting of the steps of:
i) providing at least one nucleic acid primer comprising at least one labelled nucleotide,
ii) providing enzymes and reagents for amplification of a target nucleic acid using said at least one labelled nucleic acid primer,
iii) incubating the components of the reaction under conditions suited for amplification of the target nucleic acid,
iv) separating the amplified nucleic acids according to their size,
v) detecting the amplified nucleic acids via the labelled nucleotide, wherein the nucleic acid primer consists of the following sequence:
   5'-NₐXN_{b}-3',
   wherein N may be any nucleotide,
   wherein "a" and "b" represent the number of nucleotides and may range from 4 to 17,
   wherein "a" and "b" differ by 5 or less, wherein X is said at least one labelled nucleotide and wherein the nucleic acid primer has a length of 15 to 34 nucleotides.

"Nucleic acid primer" herein means an oligonucleotide suitable for amplification. It will be understood by the person skilled in the art that for being suited for amplification the 3'-end shall be accessible for elongation by a polymerase. Nucleic acid primers may be prepared using any suitable method, such as, for example, the phosphotriester and phosphodiester methods or automated embodiments thereof. In one such automated embodiment diethylphosphoramidites are used as starting materials and may be synthesized as described by Beaucage et al., Tetrahedron Letters, 22:1859-1862 (1981). One method for synthesizing oligonucleotides on a modified solid support is described in U.S. Pat. No. 4,458, 006. It is also possible to use a primer which has been isolated from a biological source (such as a restriction endonuclease digest). The length of nucleic acid primer may vary. The length may be adapted to the needs. "Nucleic acid" in the context of the method according to the present invention means a specific class of nucleic acid, *inter alia*, RNA, DNA, cDNA (complementary DNA), LNA (locked nucleic acid), mRNA (messenger RNA), mtRNA (mitochondrial), rRNA (ribosomal RNA), tRNA (transfer RNA), nRNA (nuclear RNA), siRNA (short interfering RNA), snRNA (small nuclear RNA), snoRNA (small nucleolar RNA), scaRNA (Small Cajal Body specific RNA), microRNA, dsRNA (doubled-stranded RNA), ribozyme, riboswitch, viral RNA, dsDNA (double-stranded DNA), ssDNA (single-stranded DNA), plasmid DNA, cosmid DNA, chromosomal DNA, viral DNA, mtDNA (mitochondrial DNA), nDNA (nuclear DNA), snDNA (small nuclear DNA) or the like or any other class or sub-class of nucleic acid which is distinguishable from the bulk nucleic acid in a sample.

The inventors found that the use of internally labelled nucleic acid primers leads to a diminishing of fragments causing dye blobs. The dye blobs are especially undesirable if the amplification products have to be separated according to their lengths before detection. Hence, in the method according to the present invention step iv) comprises a step of separating the amplified nucleic acid according to their lengths, before the detection of the amplified nucleic acids via the labelled nucleotide. The person skilled in the art is aware of a great number of methods to separate nucleic acids according to their length. One commonly used method is gel electrophoresis. Hence, in one embodiment of the present invention the separation of the amplified nucleic acid according to their size is performed using gel electrophoresis. Gel electrophoresis is a technique used for the separation of nucleic acid molecules using an electric field applied to a gel matrix. The term "gel" refers to the matrix used to contain, then separate the target molecules. In most cases, the gel is a crosslinked polymer whose composition and porosity is chosen based on the specific weight/length and composition of the targets to be analyzed. When separating small nucleic acids (DNA, RNA, or oligonucleotides) the gel is usually composed of different concentrations of acrylamide and a cross-linker, producing different sized mesh networks of polyacrylamide. Acrylamid may be for example of the group of acrylamid, linear polyacrylamide, polydimethyl acrylamide or polyisopropyl acrylamide. When separating larger nucleic acids (greater than a few hundred bases), purified agarose may be chosen as the matrix. In both cases, the gel forms a solid, yet porous matrix. Agarose is composed of long unbranched chains of uncharged carbohydrate without cross links resulting in a gel with large pores allowing for the separation of macromolecules and macromolecular complexes. Hence, in one embodiment of the present invention, the matrix used for the separation of the nucleic acids is polyacrylamide or agarose.

Gel electrophoresis is used in forensics, molecular biology, genetics, microbiology and biochemistry. The results can be analyzed quantitatively by visualizing labelled nucleic acids in the gel with an imaging device, usually comprising a light source to excite fluorescence of the labels, e.g. by a laser. The signal from the label is recorded with a detection device, and the intensity of the bands or spots are measured. The measurement and analysis are mostly done with specialized software which transforms the detected signals according to the length of the nucleic acid and the amount of detected nucleic acids into an electropherogram. In a preferred embodiment of the present invention the separation of the amplified nucleic acids is performed by capillary gel electrophoresis.

Furthermore, the person skilled in the art knows methods to label an oligonucleotide at an internal position. One possibility is the attachment of the label to the nucleobase, e.g. an exocyclic amino group (also referred herein as amine group) of the nucleobase, another possibility is the attachment of the label to the 2'-carbon of the sugar.

"Exocyclic" refers to a group situated outside of the ring of a cyclic chemical structure, e.g. a portion of a substituent of the ring is exocyclic to the ring. As used herein, exocyclic amine refers to an amine group that is a substituent of a ring of a heterocyclic base and includes embodiments in which the nitrogen of the amine group is attached directly to a member of the ring structure and also includes embodiments in which the nitrogen of the amine group may be linked to the ring structure of the heterocyclic base via an intervening group.

Furthermore, it is possible to link the label to a 2'-modified nucleotide, such as, for example, the amine group is attached directly to the 2'-carbon of the sugar or the nitrogen of the amine group may be linked to the 2'-carbon of the sugar via an intervening group.

In one embodiment of the present invention the label is linked to said labelled nucleotide (X) via the exocyclic amine group or a modified 2'-postion of the labelled nucleotide (X). In a preferred embodiment the label is linked to the exocyclic amino group of the labelled nucleotide (X). In a preferred embodiment the label is linked to an exocyclic amine group of a thymine.

Possibilities to label an oligonucleotide are known to the skilled person and comprise but are not limited to amino-modifier-C6'-dT CEP (such as 5-[N-(trifluoroacetylaminohexyl)-3-acrylimido], also known as 5-[E-2-[N-[6-(trifluoroacetamido)hexyl]carboxamido]vinyl]), amino-modifier-C2-dT CEP, 5-aminoallyl-dU CEP, amino-modifier-C6-dA CEP, amino-modifier-C6-dG CEP (such as 3'-O-[(diisopropylamino)(2-cyanoethoxy)phosphino]-5'-O-(4,4'-dimethoxytrityl)-N2-[(dimethylamino) methylidene]-N8-[6-(trifluoroacetylamino)hexyl]-2'-deoxyguanosine), amino-modifier-15-dT CEP (such as 3'-O-[(diisopropylamino)(2-cyanoethoxy)phosphino]-5'-O-(4,4'-dimethoxytrityl)-5-[E-2'-[N-[15-(trifluoroacetamido)-7-aza-10,13-dioxa-8-oxopentadecyl]carboxamido]vinyl]-2'-deoxyuridine, 2'-O-aminolinker-U CEP (such as 3'-O-[(diisopropylamino)(2-cyanoethoxy)phosphino]-5'-O-(4,4'-dimethoxytrityl)-2'-O-2-[2-(trifluoroacetamido)ethoxy]ethyluridine), and 2'-amino-D-uridine.

The position of the labelled nucleotide (X) within the nucleic acid primer may vary. However, as the inventors found that a nucleic acid primer which is internally labelled diminishes the appearance of dye blobs in electropherograms, the nucleic acid primer according to the present invention is internally labelled, i.e. the labelled nucleotide is not the nucleotide at the 5'- or 3'-position of the nucleic acid primer. The value of "a" and between 4 and 17. The inventors unexpectedly found that the effect of diminishing of dye blobs is enhanced the more the labelled nucleotide is in a central position of the primer. Hence, "a" and "b" differ by by 5 or less. In a further preferred embodiment "a" and "b" have the same value. The inventors found the greatest effect with respect to diminishing of the dye blobs is reached when "a" and "b" are 10. Hence, in one embodiment of the present invention "a" and "b" are 10. However, the skilled artisan may change the exact position of the label dependent on the length and sequence of the primer. It will be understood by those of ordinary skills in the art that the nucleotides or modified nucleotides (N) do not necessarily have to be the same over the whole molecule, e.g. if "a" or "b" are 5 this does not mean that in the resulting sequence NNNNNXNNNNN that all "N" are the same nucleotides. Moreover, the skilled artisan will choose the nucleotides or modified nucleotides of the nucleic acid primer according to the target nucleic acid to be amplified, i.e. he will choose a sequence complementary to a part of the target nucleic acid.

The length of the nucleic acid primer may be adapted according to the needs and depending on the desired specificity, e.g. longer primers have a higher degree of specificity. In the present invention, the nucleic acid primer has a total length between 15 and 34 nt.

The technical effect achieved by the method according to the present invention may be observed over a great variety of labels. Hence, it may in principle be performed with any label suited to label nucleic acids. However, in one embodiment of the present invention the label is selected from the group of labels consisting of fluorophores, chromophores, radioisotopes, chemiluminescence and enzymes. In a preferred embodiment the label is a fluorophore, preferably selected from the group of fluorophores comprising 5- or 6-carboxyfluorescein (FAM™), VIC™, NED™, fluorescein, fluorescein isothiocyanate (FITC), IRD-700/800, cyanine dyes, such as CY3™, CY5™, CY3.5™, CY5.5™, Cy7™, xanthen, 6-carboxy-2',4',7',4,7-hexachlorofluorescein (HEX), 6-carboxy-1,4-dichloro-2',7'-dichloro-fluorescein (TET®), 6-carboxy-4',5'-dichloro-2',7'-dimethodyfluorescein (JOE™), N,N,N',N'-tetramethyl-6-carboxyrhodamine (TAMRA™), 6-carboxy-X-rhodamine (ROX), 5-carboxyrhodamine-6G (R6G5), 6-carboxyrhodamine-6G (RG6), rhodamine, rhodamine green, rhodamine red, rhodamine 110, Rhodamin 6G®, BODIPY dyes, such as BODIPY TMR, oregon green, coumarines, such as umbelliferone, benzimides, such as Hoechst 33258; phenanthridines, such as Texas Red®, California Red®, Yakima Yellow, Alexa Fluor® 350, Alexa Fluor® 405, Alexa Fluor® 430, Alexa Fluor® 488, Alexa Fluor® 500, Alexa Fluor® 514, Alexa Fluor®532, Alexa Fluor® 546, Alexa Fluor® 555, Alexa Fluor® 568, Alexa Fluor® 594, Alexa Fluor® 610, Alexa Fluor® 633, Alexa Fluor® 647, Alexa Fluor® 660, Alexa Fluor® 680, Alexa Fluor® 700, Alexa Fluor® 750, PET®, ethidium bromide, acridinium dyes, carbazol dyes, phenoxazine dyes, porphyrine dyes, polymethin dyes, Atto 390, Atto 425, Atto 465, Atto 488, Atto 495, Atto 520, Atto 532, Atto 550, Atto 565, Atto 590, Atto 594, Atto 620, Atto 633, Atto 647N, Atto 655, Atto RhoG6, Atto Rho11, Atto Rho12, Atto Rho101, BMN™-5, BMN™-6, CEQ8000 D2, CEQ8000 D3, CEQ8000 D4, DY-480XL, DY-485XL, DY-495, DY-505, DY-510XL, DY-521XL, DY-521XL, DY-530, DY-547, DY-550, DY-555, DY-610, DY-615, DY-630, DY-631, DY-633, DY-635, DY-647, DY-651, DY-675, DY-676, DY-680, DY-681, DY-700, DY-701, DY-730, DY-731, DY-732, DY-750, DY-751, DY-776, DY-780, DY-781, DY-782, 6-carboxy-4',5'-dichloro-2',7'-dimethoxyfluorescein (JOE), TET™, CAL Fluor® Gold 540, CAL Fluor RED 590, CAL Fluor Red 610, CAL Fluor Red 635, IRDye® 700Dx, IRDye® 800CW, Marina Blue®, Pacific Blue®, Yakima Yellow®, 6-(4,7-Dichloro-2',7'-diphenyl-3',6'-dipivaloylfluorescein-6-carboxamido)-hexyl-1-O-(2-cyanoethyl)-(N,N-diisopropyl)-phosphoramidite (SIMA), CAL Fluor® Gold 540, CAL Fluor® Orange 560, CAL Fluor Red 635, Quasar 570, Quasar 670, LIZ, Sunnyvale Red, LC Red® 610, LC Red® 640, LC Red®670, and LC Red® 705. In a further preferred embodiment of the present invention the label is selected from the group of fluorophores consisting of Atto 465, DY-485XL, FAM™, Alexa Fluor® 488, DY-495, Atto 495, DY-510XL, JOE, TET™, CAL Fluor® Gold 540, DY-521XL, Rhodamin 6G®, Yakima Yellow®, Atto 532, Alexa Fluor®532, HEX, SIMA, Atto RhoG6, VIC, CAL Fluor Orange 560, DY-530, TAMRA™, Quasar 570, Cy3™, NED™, DY-550, Atto 550, Alexa Fluor® 555, PET®, CAL Fluor RED 590, ROX, Texas Red®, CAL Fluor Red 610, CAL Fluor Red 635, Atto 633, Alexa Fluor® 633, DY-630, DY-633 ,DY-631, LIZ, Quasar 670, DY-635, and Cy5™. In a yet further preferred embodiment the label is selected from group of fluorophores consisting of FAM™, DY-510XL, DY-530, and Atto 550.

In one embodiment the nucleic acid primer used in the method of the present invention comprise modified nucleotides. Modified nucleotides encompass nucleotides that are labelled nucleotides selected from the group consisting of nucleotides comprising a label as outlined herein above, modified nucleotides, abasic sites and quenchers. The skilled artisan is able to choose suitable nucleotides out of a comprehensive number of different nucleotide types according to the needs. In a preferred embodiment each N is independently selected from the group consisting of thymidine, adenosine, guanosine, cytidine, uridine and inosine. Nucleotide bases are also encompassed, wherein the nucleotide bases are for example hypoxanthine, xanthine, 7-methylguanine, xanthinosine, 7-methylguanosine, 5,6-dihydrouracil, 5-methylcytosine, pseudouridine, dihydrouridine, 5-methylcytidine. Hence, in one embodiment one or more "N" is a modified nucleotide.

"Modified nucleotides" herein refers to non natural nucleotides. "Modified nucleotides" are known by the skilled artisan. However, in one embodiment of the present invention modified nucleotides are selected from the group consisting of locked nucleic acid (LNA) or peptide nucleic acid (PNA), labelled nucleotides as outlined herein above, nucleotides linked to a quencher, and abasic sites.

"Quenching" refers to any process which decreases the fluorescence intensity of a given substance. "Quencher" in context of the present invention relates to a residue which is suited for quenching, e.g. the basic fluorescence of the label without activation. A variety of processes can result in quenching, such as excited state reactions, energy transfer, complex-formation and collisional quenching. As a consequence, quenching is often heavily dependent on pressure and temperature. Molecular oxygen and the iodide ion are common chemical quenchers. Quenching poses a problem for non-instant spectroscopic methods, such as laser-induced fluorescence. Quenching is the basis for Fluorescence Resonance Energy Transfer (FRET) assays. Quenching and dequenching upon interaction with a specific molecular biological target is the basis for activatable optical contrast agents for molecular imaging. There are a few distinct mechanisms by which energy can be transferred: non-radiatively (without absorption or emission of photons) between two dyes, a donor and an acceptor. Förster resonance energy transfer, Dexter energy transfer, Exciplex (excited state complex) formation, and static quenching (also referred to as contact quenching). The skilled artisan is able to choose quenchers according to the needs.

An "abasic site" (also known as AP site (apurinic/apyrimidinic site)) in context of the present invention is a location in a nucleic acid/oligonucleotide that has neither a purine nor a pyrimidine base. Abasic sites have been mentioned in "Compositions and Methods for Enhancing Hybridization and Priming Specificity" (US-B1 6,361,940) as well as in "Method for Label-free Detection of Hybridized DNA Target" (US-B1 6,579,680) and "Use of Abasic Site-Containing DNA Strands for Nucleobase Recognition in Water" Yoshimoto et al., JACS Communications, published on Web, 07/04/2003.

The skilled artisan will furthermore recognize that the nucleic acid primer according to the present invention may consist of different types of nucleic acids or modified nucleic acids. Hence, in a preferred embodiment the nucleic acid primer consists of DNA, RNA, PNA, or LNA.

The inventors unexpectedly found that the technical effect of the method according to the present invention can be further enhanced if the label is attached to the nucleotide within the nucleic acid primer by a linker. Hence, in one embodiment the label is attached to the nucleotide via a linker. Suitable linkers may comprise a spacer as known from the prior art. For example linkers comprising 2 to 18 carbon atoms like for example a C₅-linker or a C₆-linker may be used between the label and the nucleotide according to the present invention. Preferably the spacer is a branched or an unbranched aminolinker. In a further preferred embodiment the spacer is selected from the group of C₂- to C₁₈- linker or aminolinker, preferably the spacer is from the group of C₃-to C₁₂-linker or aminolinker. The linker or aminolinker is preferably a linear alkyl. In a very special embodiment the spacer is a C₅-spacer or a C₆-spacer. Aminolinkers may be used to incorporate a primary amino group onto the 5'-end, the 3'-end, the exocyclic amino group or a modified 2'-position of an oligonucleotide. The active amino group may be used for labelling oligonucleotides using labelling agents, e g. in the form of N-hydroxy succinimide ester.

The method of the present invention comprises amplification of nucleic acids. The amplification may be performed by a variety of known amplification methods. Hence, in one embodiment of the present invention the amplification is performed by a method selected from the group consisting of polymerase chain reaction (PCR), isothermal amplification (such as in Walker, et al., "Strand displacement amplification--an isothermal, in vitro DNA amplification technique," Nucleic Acids Res. 20(7):1691-6 (1992)), ligase chain reaction (LCR; such as in Landegren, et al., "A Ligase-Mediated Gene Detection Technique," Science 241:1077-1080, 1988, or, in Wiedmann, et al., "Ligase Chain Reaction (LCR)--Overview and Applications," PCR Methods and Applications (Cold Spring Harbor Laboratory Press, Cold Spring Harbor Laboratory, NY, 1994) pp. S51-S64.)), transcription-based amplification system (TAS), nucleic acid sequence based amplification (NASBA; Kievits et al. (1991) J Virol Methods 35:273-286),, rolling circle amplification (RCA; such as in Liu, et al., "Rolling circle DNA synthesis: Small circular oligonucleotides as efficient templates for DNA polymerases," J. Am. Chem. Soc. 118:1587-1594 (1996)), transcription-mediated amplification (TMA; Vuorinen et al. (1995) J Clin Microbiol 33: 1856-1859), self-sustaining sequence replication (3SR), Qβ amplification, strand displacement amplification (SDA) (Walker et al. (1992) Nucleic Acids Res 20(7):1691-6), multiple displacement amplification (MDA) (Dean et al. (2002) Proc Natl Acad Sci USA 99(8): 5261-5266), restriction aided RCA (Wang et al. (2004) Genome Res 14:2357-2366), single primer isothermal amplification (SPIA; Dafforn et al. (2004) Biotechniques 37(5):854-7), loop mediated isothermal amplification (LAMP; Notomi et al. (2000) Nucleic Acids Res 28(12):e63), transcription mediated amplification (TMA), helicase-dependent amplification (HDA), thermostable HDA (tHDA) (An et al. (2005) J Biol Chem 280(32):28952-28958), smart-amplification process (SMAP; Mitani et al. (2007) Nat Methods 4(3):257-62)), quantitative real-time PCR (qPCR), reverse-transcriptase PCR (RT-PCR), Sanger-Sequencing.

The present invention furthermore relates to the use of a nucleic acid primer in a method according to claims 1 to 6, wherein said nucleic acid primer consists of the following sequence:
5'-NₐXN_{b}-3',
   wherein N may be any nucleotide,
   wherein "a" and "b" represent the number of nucleotides and may range from 4 to 17,
   wherein "a" and "b" differ by 5 or less,
   wherein X is said at least one labelled nucleotide
   and wherein said nucleic acid primer has a length of 15 to 34 nucleotides.

As outlined above, the amplification method may be chosen from different methods. Depending on the template nucleic acid and the method of amplification different enzymes or reagents may be used according to the present invention. The DNA polymerase is preferably "thermostable", meaning that it is generally stable at the high temperatures used for denaturation of DNA strands. More particularly, the thermostable DNA polymerases are not substantially inactivated at the high temperatures used in PCR. Such temperatures will vary depending upon a number of reaction conditions, including pH, salt concentration, and other conditions known in the art. In context of the invention, a preferred embodiment involves a thermostable DNA polymerase, which may harbour a 5'-3' exonuclease activity or a thermostable DNA polymerase with reduced or free of 5'-3' exonuclease activity. In another embodiment, the thermostable DNA polymerase may additionally harbour, a 3'-5' exonuclease (proof-reading) activity. A number of thermostable DNA polymerases have been reported in the art. Particularly useful polymerases are those obtained from various Thermus bacterial species, such as Thermus aquaticus, Thermus filiformis, Thermus flavus or Thermus thermophilus. Other useful thermostable polymerases are obtained from a variety of other microbial sources including Thermococcus literalis, Pyrococcus furiosus, Thermotoga sp. and those described in WO-A-89/06691 (published July 27, 1989). Such polymerases include, but are not limited to, Thermus thermophilus (Tth) DNA polymerase, Thermus aquaticus (Taq) DNA polymerase, Thermotoga neopalitana (Tne) DNA polymerase, Thermotoga maritima (Tma) DNA polymerase, Thermococcus litoralis (Tli or VENT.(TM).) DNA polymerase, Thermus eggertssonii (Teg) DNA polymerase, Pyrococcus furiosus (Pfu) DNA polymerase, DEEPVENT. DNA polymerase, Pyrococcus woosii (Pwo) DNA polymerase, Pyrococcus sp KDD2 (KOD) DNA polymerase, Bacillus sterothermophilus (Bst) DNA polymerase, Bacillus caldophilus (Bea) DNA polymerase, Sulfolobus acidocaldarius (Sac) DNA polymerase, Thermoplasma acidophilum (Tac) DNA polymerase, Thermus flavus (Tfl/Tub) DNA polymerase, Thermus ruber (Tru) DNA polymerase, Thermus brockianus (DYNAZYME) DNA polymerase, Methanobacterium thermoautotrophicum (Mth) DNA polymerase, mycobacterium DNA polymerase (Mtb, Mlep), and mutants, variants and derivatives thereof including hot-start polymerases, which are inactive at room temperature and where full activity can fast be regained by an incubation at elevated temperature.

As used herein, the term "dNTP" refers to desoxyribonucleoside triphosphates. Non-limiting examples of such dNTPs are dATP, dGTP, dCTP, dTTP, dUTP, which may also be present in the form of labelled derivatives, for instance comprising a fluorescence label, a radioactive label, a biotin label. dNTPs with modified nucleotide bases are also encompassed, wherein the nucleotide bases are for example hypoxanthine, xanthine, 7-methylguanine, inosine, xanthinosine, 7-methylguanosine, 5,6-dihydrouracil, 5-methylcytosine, pseudouridine, dihydrouridine, 5-methylcytidine. Furthermore, ddNTPS of the above-described molecules are encompassed in the present invention.

As used herein, the term "NTP" refers to ribonucleoside triphosphates. Non-limiting examples of such NTPs are ATP, GTP, CTP, TTP, UTP, which may also be present in the form of labelled derivatives, for instance comprising a fluorescent label, a radioactive label, a biotin label.

The embodiments of the nucleic acid primer described herein above apply also for the embodiments of the used nucleic acid primer.

### EXAMPLES

### Example 1

The PCR in these examples was carried out as follows:
0.2 µl Multi Taq2 DNA Polymerase (QIAGEN, Hilden, Germany) was used as Hot-start DNA polymerase. 2.5 µl Reaction MixB (QIAGEN, Hilden, Germany) was used as a PCR buffering solution. 0.4 µM internally labelled forward primer D16-P-intern (5'-gggggtctaagagcXtgtaaaaag-3'; SEQ ID NO. 1; wherein X represents ATTO550 linked to the exocyclic amino group of a thymidine nucleotide) or 0.4 µM labelled forward primer D16-P-ATTO550 (5'-ATTO550-gggggtctaagagcttgtaaaaag-3'; SEQ ID NO. 2) in combination with 0.4 µM unlabelled D16_rev reverse primer (5'-gtttgtgtgtgcatctgtaagcatgtatc-3'; SEQ ID NO. 3), all produced by Biomers.net (Ulm, Germany), were used for PCR performance.

**Table 1:**

| SEQ ID NO. | Name | Sequence | Annotations |
|---|---|---|---|
| 1 | D16-P-intern (internally labelled forward primer) | 5'-gggggtctaagagcXtgtaaaaag-3' | X represents ATTO550 linked to the exocyclic amino group of a thymidine nucleotide |
| 2 | D16-P-ATTO550 (labelled forward primer) | 5'-ATTO550-gggggtctaagagcttgtaaaaag-3' | standard PCR primer |
| 3 | D16_rev (unlabelled reverse primer) | 5'-gtttgtgtgtgcatctgtaagcatgtatc-3' | |
| 4 | EPL-11-Y-intern1 (internally labelled forward primer) | 5'-aaXgaattgaacaaatgagtgagtg-3' | X represents ATTO550 linked to the exocyclic amino group of a thymidine nucleotide |
| 5 | EPL-11-Y-intern2 (internally labelled forward primer) | 5'-aatgaaXtgaacaaatgagtgagtg-3' | X represents ATTO550 linked to the exocyclic amino group of a thymidine nucleotide |
| 6 | EPL-11-Y-intern3 (internally labelled forward primer) | 5'-aatgaatXgaacaaatgagtgagtg-3' | X represents ATTO550 linked to the exocyclic amino group of a thymidine nucleotide |
| 7 | EPL-11-Y-intern4 (internally labelled forward | 5'-aatgaattgaacaaaXgagtgagtg-3' | X represents ATTO550 linked to the exocyclic amino group of a |
| | primer) | | thymidine nucleotide |
| 8 | EPL-11-Y-intern5 (internally labelled forward primer) | 5'-aatgaattgaacaaatgagXgagtg-3' | X represents ATTO550 linked to the exocyclic amino group of a thymidine nucleotide |
| 9 | EPL-11-Y-intern 6 (internally labelled forward primer) | 5'-aatgaattgaacaaatgagtgagXg-3' | X represents ATTO550 linked to the exocyclic amino group of a thymidine nucleotide |
| 10 | EPL11/2 (unlabelled reverse primer) | 5'-gaacaactctggttgtattgtcttc-3' | |

The volume of 25 µl was completed by Nuclease-free water (QIAGEN, Hilden, Germany). PCR was carried out using a thermocycler 9700 PCR System Gold (Life Technologies Corporation, Carlsbad, CA, USA) with the following protocol:

**Table 2: Cycling conditions for example 1**

| **Temperature** | **Time** | | |
|---|---|---|---|
| 96°C | 2 min | | |
| 94°C | 30 s | 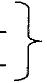 | 30 cycles |
| 59°C | 120 s | | |
| 72°C | 90 s | | |
| 60°C | 45 min | | |
| 4°C | ∞ | | |

1 µl of cycled PCR probes were added to 12 µl Hi-Di Formamid (Life Technologies Corporation, Carlsbad, CA, USA) and 0.5 µl DNA size standard 550 (BTO) (QIAGEN, Hilden, Germany) and incubated for 3 min at 95°C.
The denatured PCR probes were analyzed by capillary electrophoresis using a 3500 Genetic Analyzer (Life Technologies Corporation, Carlsbad, CA, USA) under standard conditions.

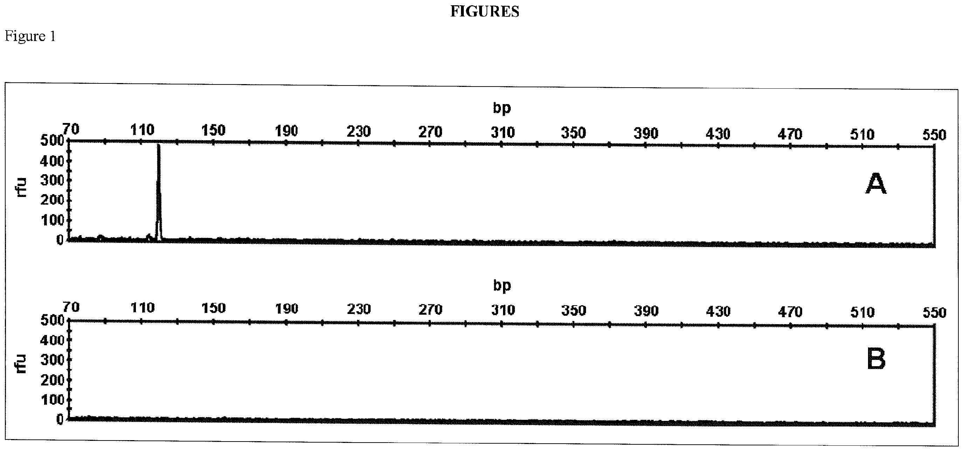

The results shown in Figure 1 clearly demonstrate that the use of internally labelled primers result in a reduction of artificial unwanted peaks of ordinary background noise. The baseline in the range of 70 to 550 bp is absolutely clean, i.e. without any background peaks or dye blobs. In contrast, standard PCR primers that are labelled at the 5' end, cause numerous artificial peaks with up to 500 relative fluorescence units (rfu). PCR amplifications with minimal amounts of template DNA result typically in PCR products giving peaks of around 200 rfu. Using standard labelled primers the interpretation of electropherograms is impaired by numerous, high, artificial peaks (dye blobs), which results in an incomplete appraisement of the probe. Using internal labelled primers, PCR products of 200 rfu can be clearly distinguished and assigned as no dyeblobs are visible. These improvements give a huge advantage in the sensitivity of the method.

### Example 2

When using primers that are labelled at any internally base a strong reduction of background noise is apparent. However, in further experiments it is shown that the effect can be further enhanced by choosing the position for the internal label within the primer. Primers are labelled at differing base pair positions of the sequence of EPL-11-Y (SEQ ID NO. 4 to 9; see Table 1). The analysis is performed as described in Example 1 using the primer EPL11/2 (5'-gaacaactctggttgtattgtcttc-3'; SEQ ID NO. 10) as the reverse primer and following cycling conditions.

**Table 3: Cycling conditions for example 2**

| **Temperature** | **Time** | | |
|---|---|---|---|
| 94°C | 4 min | | |
| 96°C | 30 s | 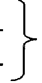 | 5 cycles |
| 61°C | 120 s | | |
| 72°C | | | |
| 94°C | 30 s | 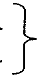 | 25 cycles |
| | 120 s | | |
| 72°C | 75 s | | |
| 68°C | 60 min | | |
| 10°C | ∞ | | |

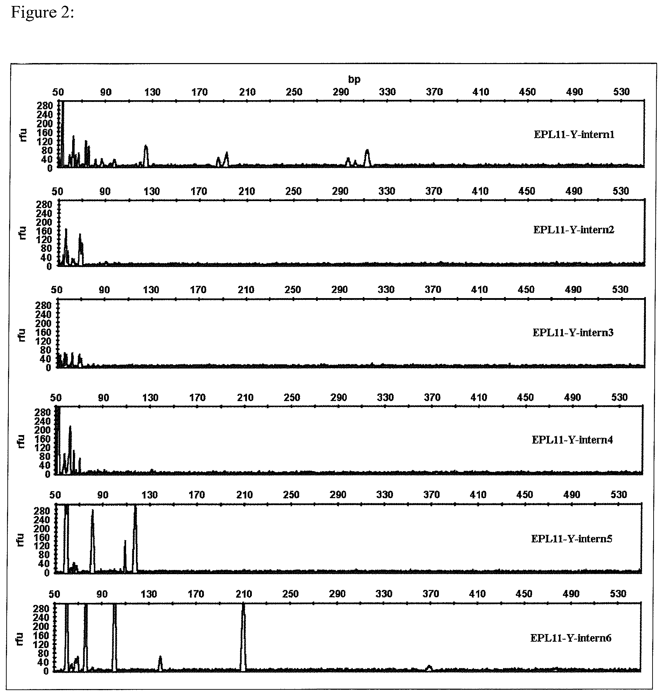

The results shown in Figure 2 demonstrate that the effect of baseline smoothing can be further enhanced by choosing an internal position for the label that is close to the middle position of the primer.

### Figure legend:

**Figure 1****: Baseline improvement by internally labelled primer.** A PCR without template DNA was performed with each 0.4 µM forward and reverse primer. The PCR probes were analyzed by capillary electrophoresis afterwards. In the upper electropherogram the result of the no-template-control (NTC) PCR with a standard labelled primer is shown (A). The result of the NTC-PCR using the internally labelled primer is shown in B. The internally labelled primer does not causes any false positive signals in the range between 70 and 550 bp, the baseline is absolutely flat.
**Figure 2****: Influence of the internal dye coupling position. The primer EPL11-Y has been** labelled fluorescently at various internal primer positions (s. Table 1). Results of capillary electrophoresis after PCR with 0.4 µM primer (final concentration), but without template DNA are shown. The further the dye is coupled to the middle of the primer, the fewer artificial peaks occur.

## Claims

1. A method for sequencing, or amplifying and detecting nucleic acids consisting of the steps of:
i) providing at least one nucleic acid primer comprising at least one labelled nucleotide,
ii) providing enzymes and reagents for amplification of a target nucleic acid using said at least one labelled nucleic acid primer,
iii) incubating the components of the reaction under conditions suited for amplification of the target nucleic acid,
iv) separating the amplified nucleic acids according to their size,
v) detecting the amplified nucleic acids via the labelled nucleotide, wherein the nucleic acid primer consists of the following sequence:
5'-NₐXN_{b}-3',
wherein N may be any nucleotide,
wherein "a" and "b" represent the number of nucleotides and may range from 4 to 17,
wherein "a" and "b" differ by 5 or less,
wherein X is said at least one labelled nucleotide
and wherein the nucleic acid primer has a length of 15 to 34 nucleotides.

2. The method according to claim 1, wherein the label is linked to said nucleotide via the exocyclic amino group or 2'-amino group of the nucleotide.

3. The method according to any one of claims 1 to 2, wherein the label is selected from the group of labels consisting of fluorophores, chromophores, radioisotopes, and chemiluminescent, and the group of fluorophores comprising 5- or 6-carboxyfluorescein (FAM™), VIC™, NED™, fluorescein, fluorescein isothiocyanate (FITC), IRD-700/800, cyanine dyes, such as CY3™, CY5™, CY3.5™, CY5.5™, Cy7™, xanthen, 6-carboxy-2',4',7',4,7-hexachlorofluorescein (HEX), 6-carboxy-1,4-dichloro-2',7'-dichloro-fluorescein (TET®), 6-carboxy-4',5'-dichloro-2',7'-dimethodyfluorescein (JOE™), N,N,N',N'-tetramethyl-6-carboxyrhodamine (TAMRA™), 6-carboxy-X-rhodamine (ROX), 5-carboxyrhodamine-6G (R6G5), 6-carboxyrhodamine-6G (RG6), rhodamine, rhodamine green, rhodamine red, rhodamine 110, Rhodamin 6G®, BODIPY dyes, such as BODIPY TMR, oregon green, coumarines, such as umbelliferone, benzimides, such as Hoechst 33258; phenanthridines, such as Texas Red®, California Red®, Yakima Yellow, Alexa Fluor® 350, Alexa Fluor® 405, Alexa Fluor® 430, Alexa Fluor® 488, Alexa Fluor® 500, Alexa Fluor® 514, Alexa Fluor® 532, Alexa Fluor® 546, Alexa Fluor® 555, Alexa Fluor® 568, Alexa Fluor® 594, Alexa Fluor® 610, Alexa Fluor® 633, Alexa Fluor® 647, Alexa Fluor® 660, Alexa Fluor® 680, Alexa Fluor® 700, Alexa Fluor® 750, PET®, ethidium bromide, acridinium dyes, carbazol dyes, phenoxazine dyes, porphyrine dyes, polymethin dyes, Atto 390, Atto 425, Atto 465, Atto 488, Atto 495, Atto 520, Atto 532, Atto 550, Atto 565, Atto 590, Atto 594, Atto 620, Atto 633, Atto 647N, Atto 655, Atto RhoG6, Atto Rho11, Atto Rho12, Atto Rho101, BMN™-5, BMN™-₆, CEQ8000 D2, CEQ8000 D3, CEQ8000 D4, DY-480XL, DY-485XL, DY-495, DY-505, DY-510XL, DY-521XL, DY-521XL, DY-530, DY-547, DY-550, DY-555, DY-610, DY-615, DY-630, DY-631, DY-633, DY-635, DY-647, DY-651, DY-675, DY-676, DY-680, DY-681, DY-700, DY-701, DY-730, DY-731, DY-732, DY-750, DY-751, DY-776, DY-780, DY-781, DY-782, 6-carboxy-4',5'-dichloro-2',7'-dimethoxyfluorescein (JOE), TET™, CAL Fluor® Gold 540, CAL Fluor RED 590, CAL Fluor Red 610, CAL Fluor Red 635, IRDye® 700Dx, IRDye® 800CW, Marina Blue®, Pacific Blue®, Yakima Yellow®, 6-(4,7-Dichloro-2',7'-diphenyl-3',6'-dipivaloylfluorescein-6-carboxamido)-hexyl-1-O-(2-cyanoethyl)-(N,N-diisopropyl)-phosphoramidite (SIMA), CAL Fluor® Gold 540, CAL Fluor® Orange 560, CAL Fluor Red 635, Quasar 570, Quasar 670, LIZ, Sunnyvale Red, LC Red® 610, LC Red® 640, LC Red® 670, and LC Red® 705.

4. The method according to any one of claims 1 to 3, wherein the amplification method is selected from the group consisting of polymerase chain reaction (PCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR) and Qβ amplification strand displacement amplification (SDA), multiple displacement amplification (MDA), loop mediated isothermal amplification (LAMP), helicase-dependent amplification (HDA), smart-amplification process (SMAP), quantitative real-time PCR (qPCR), reverse-transcriptase PCR (RT-PCR), Sanger-Sequencing.

5. Use of a nucleic acid primer in a method according to claims 1 to 4, wherein said nucleic acid primer consists of the following sequence:
5'-NₐXN_{b}-3',
wherein N may be any nucleotide,
wherein "a" and "b" represent the number of nucleotides and may range from 4 to 17, wherein "a" and "b" differ by 5 or less,
and wherein X is said at least one labelled nucleotide
and wherein said nucleic acid primer has a length of 15 to 34 nucleotides.

## Patentansprüche

1. Ein Verfahren zur Sequenzierung, oder Amplifikation und Detektion von Nukleinsäuren bestehend aus den Schritten:
i) bereitstellen von mindestens einem Nukleinsäure-Primer, der mindestens ein markiertes Nukleotid umfasst,
ii) bereitstellen von Enzymen und Reagenzien für die Amplifikation von Ziel-Nukleinsäuren unter Verwendung von genanntem mindestens einem markiertem Nukleinsäure-Primer,
iii) inkubieren der Bestandteile der Reaktion bei Bedingungen, die für die Amplifikation der Ziel-Nukleinsäure geeignet sind,
iv) trennen der amplifizierten Nukleinsäuren nach ihrer Größe
v) detektieren der amplifizierten Nukleinsäuren durch die markierten Nukleotide,
wobei der Nukleinsäure-Primer aus folgender Sequenz besteht:
5'-NₐXN_{b}-3',
wobei N jedes Nukleotid sein kann,
wobei "a" und "b" die Anzahl an Nukleotiden darstellen und von 4 bis 17 reichen können,
wobei "a" und "b" sich durch 5 oder weniger unterscheiden,
wobei X besagtes mindestens ein markiertes Nukleotid ist
und wobei der Nukleinsäure-Primer eine Länge von 15 bis 34 Nukleotide hat.

2. Das Verfahren gemäß Anspruch 1, wobei die Markierung durch die exo-zyklische Aminogruppe oder 2'-Aminogruppe des Nukleotids, an besagtes Nukleotid gekoppelt ist.

3. Das Verfahren gemäß einem der Ansprüche 1 bis 2,
wobei die Markierung ausgewählt ist aus der Gruppe von Markierungen der Fluorophore, Chromophore, Radioisotope, und Chemilumineszenz, und der Gruppe von Fluorophoren umfassend 5- oder 6- Carboxy-Fluoreszein (FAM™), VIC™, NED™, Fluoreszein, Fluoreszein-Isothio-Zyanat (FITC), IRD-700/800, Zyanat-Farbstoffe, wie CY3™, CY5™, CY3.5™, CY5.5™, Cy7™, Xanthen, 6-Carboxy-2',4',7',4,7-hexachloro-Fluoreszein (HEX), 6-Carboxy-1,4-dichloro-2',7'-dichloro-Fluoreszein (TET®), 6-Carboxy-4',5'-dichloro-2',7'-dimethody-Fluoreszein (JOE™), N,N,N',N'-tetramethyl-6-arboxy-Rhodamin (TAMRA™), 6-Carboxy-X-Rhodamin (ROX), 5-Carboxyrhodamin-6G (R6G5), 6-Carboxyrhodamin-6G (RG6), Rhodamin, Rhodamin grün, Rhodamin rot, Rhodamin 110, Rhodamin 6G®, BODIPY- Farbstoffe wie BODIPY TMR, Oregon grün, Coumarine wie Umbelliferon, Benzimide wie Hoechst 33258; Phenanthridine, wie Texas Red®, California Red®, Yakima Yellow, Alexa Fluor® 350, Alexa Fluor® 405, Alexa Fluor® 430, Alexa Fluor® 488, Alexa Fluor® 500, Alexa Fluor® 514, Alexa Fluor® 532, Alexa Fluor® 546, Alexa Fluor® 555, Alexa Fluor® 568, Alexa Fluor® 594, Alexa Fluor® 610, Alexa Fluor® 633, Alexa Fluor® 647, Alexa Fluor® 660, Alexa Fluor® 680, Alexa Fluor® 700, Alexa Fluor® 750, PET®, Ethidiumbromid, Acridin-Farstoffe, Carbazol-Farbstoffe, Phenoxazin-Farbstoffe, Porphyrin-Farstoffe, Polymethin-Farbstoffe, Polymethin-Farbstoffe, Atto 390, Atto 425, Atto 465, Atto 488, Atto 495, Atto 520, Atto 532, Atto 550, Atto 565, Atto 590, Atto 594, Atto 620, Atto 633, Atto 647N, Atto 655, Atto RhoG6, Atto Rho11, Atto Rho12, Atto Rho101, BMN™-5, BMN™-6, CEQ8000 D2, CEQ8000 D3, CEQ8000 D4, DY-480XL, DY-485XL, DY-495, DY-505, DY-510XL, DY-521XL, DY-521XL, DY-530, DY-547, DY-550, DY-555, DY-610, DY-615, DY-630, DY-631, DY-633, DY-635, DY-647, DY-651, DY-675, DY-676, DY-680, DY-681, DY-700, DY-701, DY-730, DY-731, DY-732, DY-750, DY-751, DY-776, DY-780, DY-781, DY-782, 6-Carboxy-4',5'-dichloro-2',7'-dimethoxy-Fluoreszein (JOE), TET™, CAL Fluor® Gold 540, CAL Fluor RED 590, CAL Fluor Red 610, CAL Fluor Red 635, IRDye® 700Dx, IRDye® 800CW, Marina Blue®, Pacific Blue®, Yakima Yellow®, 6-(4,7-Dichloro-2',7'-diphenyl-3',6'-dipivaloylfluoreszein-6-carboxamido)-hexy1-1-O-(2-cyanoéthyl)-(N,N-diisopropyl)-phosphoramidit (SIMA), CAL Fluor® Gold 540, CAL Fluor® Orange 560, CAL Fluor Red 635, Quasar 570, Quasar 670, LIZ, Sunnyvale Red, LC Red® 610, LC Red® 640, LC Red® 670, und LC Red® 705.

4. Das Verfahren gemäß einem der Ansprüche 1 bis 3, wobei das Amplifikationsverfahren ausgewählt ist aus der Gruppe bestehend aus Polymerase-Kettenreaktion (PCR), Ligases-Kettenreaktion (LCR), Transkriptions-basierte Amplifikationssyteme (TAS), Nukleinsäure-Sequenz-basierte Amplifikation (NASBA), Rolling Circle Amplifikation (RCA), Transkriptions-vermittelte Amplifikation (TMA), selbst-erhaltende Sequenz-Replikation (3SR) und Qβ-Amplifikation Strang-Verdrängungsamplifikation, Multiple-Displacement Amplifikation (MDA), Loopvermittelte isothermale Amplifkation (LAMP), Helikase-abhängige Amplifikation (HDA), Smart-Amplifikationsprozesse (SMAP), quantitative Real-Time PCR (qPCR), Reverse-Transkriptase PCR (RT-PCR), Sanger-Sequenzierung.

5. Verwendung eines Nukleinsäure-Primers in einem Verfahren gemäß der Ansprüche 1 bis 4, wobei besagter Nukleinsäure-Primer aus folgender Sequenz besteht:
5'-NₐXN_{b}-3',
wobei N jedes Nukleotid sein kann,
wobei "a" und "b" die Anzahl an Nukleotiden darstellen und von 4 bis 17 reichen können,
wobei "a" und "b" sich durch 5 oder weniger unterscheiden,
wobei X besagtes mindestens ein markiertes Nukleotid ist
und wobei der Nukleinsäure-primer eine Länge von 15 bis 34 Nukleotide hat.

## Revendications

1. Un procédé de séquençage ou d'amplification et de détection d'acides nucléiques qui consiste en les étapes :
i) fournir au moins une amorce d'acide nucléique comprenant au moins un nucléotide marqué,
ii) fournir des enzymes et des réactifs pour l'amplification d'un acide nucléique cible en utilisant ladite au moins une amorce d'acide nucléique marquée,
iii) incuber les composants de la réaction dans des conditions adéquates pour l'amplification de l'acide nucléique cible,
iv) séparer les acides nucléiques amplifiés selon leur taille,
v) détecter les acides nucléiques amplifiés via le nucléotide marqué,
où la sonde d'acide nucléique consiste en la séquence suivante :
5'-NₐXN_{b}-3'
où N peut être n'importe quel nucléotide,
où « a » et « b » représentent le nombre de nucléotides et peuvent varier de 4 à 17,
où « a » et « b » diffèrent de 5 ou moins,
où X est ledit au moins un nucléotide marqué
et où l'amorce d'acide nucléique a une longueur de 15 à 34 nucléotides.

2. Le procédé selon la revendication 1, dans lequel le marquer est lié audit nucléotide via le groupe amino exocyclique ou le groupe 2'-amino du nucléotide.

3. Le procédé selon l'une quelconque des revendications 1 à 2, dans lequel le marqueur est choisi du groupe de marqueurs qui consiste en fluorophores, chromophores, radioisotopes, et chemiluminescents, et le groupe des fluorophores comprenant 5- ou 6-carboxyfluorescéine (FAM™), VIC™, NED™, fluorescéine, isothiocyanate de fluorescéine (FITC), IRD-700/800, les marqueurs de cyanide, tel que CY3™, CY5™, CY3.5™, CY5.5™, Cy7™, xanthen, 6-carboxy-2',4',7',4,7-hexachlorofluorescéine (HEX), 6-carboxy-1,4-dichloro-2',7'-dichloro-fluorescéine (TET®), 6-carboxy-4',5'-dichloro-2',7'-diméthodyfluorescéine (JOE™), N,N,N',N'-tétraméthyl-6-carboxyrhodamine (TAMRA™), 6-carboxy-X-rhodamine (ROX), 5-carboxyrhodamine-6G (R6G5), 6-carboxyrhodamine-6G (RG6), rhodamine, rhodamine verte, rhodamine rouge, rhodamine 110, Rhodamin 6G®, les marqueurs BODIPY, tel que BODIPY TMR, oregon green, les coumarines, tel que umbelliferone, les benzimides, tel que Hoechst 33258; les phenanthridines, tel que Texas Red®, California Red®, Yakima Yellow, Alexa Fluor® 350, Alexa Fluor® 405, Alexa Fluor® 430, Alexa Fluor® 488, Alexa Fluor® 500, Alexa Fluor® 514, Alexa Fluor® 532, Alexa Fluor® 546, Alexa Fluor® 555, Alexa Fluor® 568, Alexa Fluor® 594, Alexa Fluor® 610, Alexa Fluor® 633, Alexa Fluor® 647, Alexa Fluor® 660, Alexa Fluor® 680, Alexa Fluor® 700, Alexa Fluor® 750, PET®, bromure d'éthidium, les marqueurs d'acridinium, les marqueurs de carbazol, les marqueurs de phénoxazine, les marqueurs de porphyrine, les marqueurs de polyméthine, Atto 390, Atto 425, Atto 465, Atto 488, Atto 495, Atto 520, Atto 532, Atto 550, Atto 565, Atto 590, Atto 594, Atto 620, Atto 633, Atto 647N, Atto 655, Atto RhoG6, Atto Rho11, Atto Rho12, Atto Rho101, BMN™-5, BMN™-6, CEQ8000 D2, CEQ8000 D3, CEQ8000 D4, DY-480XL, DY-485XL, DY-495, DY-505, DY-510XL, DY-521XL, DY-521XL, DY-530, DY-547, DY-550, DY-555, DY-610, DY-615, DY-630, DY-631, DY-633, DY-635, DY-647, DY-651, DY-675, DY-676, DY-680, DY-681, DY-700, DY-701, DY-730, DY-731, DY-732, DY-750, DY-751, DY-776, DY-780, DY-781, DY-782, 6-carboxy-4',5'-dichloro-2',7'-diméthoxyfluorescéine (JOE), TET™, CAL Fluor® Gold 540, CAL Fluor RED 590, CAL Fluor Red 610, CAL Fluor Red 635, IRDye® 700Dx, IRDye® 800CW, Marina Blue®, Pacific Blue®, Yakima Yellow®, 6-(4,7-Dichloro-2',7'-diphényl-3',6'-dipivaloylfluorescéine-6-carboxamido)-hexyl-1-O-(2-cyanoéthyl)-(N,N-diisopropyl)-phosphoramidite (SIMA), CAL Fluor® Gold 540, CAL Fluor® Orange 560, CAL Fluor Red 635, Quasar 570, Quasar 670, LIZ, Sunnyvale Red, LC Red® 610, LC Red® 640, LC Red® 670, y LC Red® 705.

4. Le procédé selon l'une quelconque des revendications 1 à 3, dans lequel le procédé d'amplification est choisie du groupe qui consiste en la réaction en chaîne par polymérase (PCR), la réaction en chaîne par ligase (LCR), le système d'amplification basé sur la transcription (TAS), l'amplification basée sur la séquence d'acide nucléique (NASBA), l'amplification par cercle roulant (RCA), l'amplification médiée par la transcription (TMA), la réplication de séquence autoportante (3SR) et l'amplification Qβ par déplacement de brin (SDA), l'amplification à déplacement multiple (MDA), l'amplification isotherme médiée par des boucles (LAMP), l'amplification hélicase-dépendante (HDA), le procédé de smart-amplification (SMAP), la PCR quantitative en temps réel (qPCR), la PCR de la transcription inverse (RT-PCR), le séquençage par la méthode de Sanger.

5. Utilisation d'une amorce d'acide nucléique dans un procédé selon les revendications 1 à 4, où ladite amorce d'acide nucléique consiste en la séquence suivante :
5'-NₐXN_{b}-3'
où N peut être n'importe quel nucléotide,
où « a » et « b » représentent le nombre de nucléotides et peuvent varier de 4 à 17,
où « a » et « b » diffèrent de 5 ou moins,
où X est ledit au moins un nucléotide marqué
et où l'amorce d'acide nucléique a une longueur de 15 à 34 nucléotides.


### SEQUENCE LISTING

<110> QIAGEN GmbH
<120> Methods for sequencing, amplification and detection of nucleic acids comprising internally labelled primer
<130> B051-0080WO1
<150> EP 11 16 5142.8
   <151> 2011-05-06
<160> 10
<170> PatentIn version 3.5
<210> 1
   <211> 24
   <212> DNA
   <213> Artificial Sequence
<220>
   <223> internally labelled forward primer
<220>
   <221> misc_binding
   <222> (15)..(15)
   <223> ATTO550 linked to the exocyclic amino group of thymidine nucleotide
<400> 1
   gggggtctaa gagcttgtaa aaag 24
<210> 2
   <211> 24
   <212> DNA
   <213> Artificial Sequence
<220>
   <223> labelled forward primer
<400> 2
   gggggtctaa gagcttgtaa aaag 24
<210> 3
   <211> 29
   <212> DNA
   <213> Artificial Sequence
<220>
   <223> unlabelled reverse primer
<400> 3
   gtttgtgtgt gcatctgtaa gcatgtatc 29
<210> 4
   <211> 25
   <212> DNA
   <213> Artificial Sequence
<220>
   <223> internally labelled forward primer (EPL-11-Y-intern1)
<220>
   <221> misc_binding
   <222> (3)..(3)
   <223> ATTO550 linked to the exocyclic amino group of a thymidine nucleotide
<400> 4
   aatgaattga acaaatgagt gagtg 25
<210> 5
   <211> 25
   <212> DNA
   <213> Artificial Sequence
<220>
   <223> internally labelled forward primer (EPL-11-Y-intern2)
<220>
   <221> misc_binding
   <222> (7)..(7)
   <223> ATTO550 linked to the exocyclic amino group of thymidine nucleotide
<400> 5
   aatgaattga acaaatgagt gagtg 25
<210> 6
   <211> 25
   <212> DNA
   <213> Artificial Sequence
<220>
   <223> internally labelled forwared primer (EPL-11-Y-intern3)
<220>
   <221> misc_binding
   <222> (8)..(8)
   <223> ATTO550 linked to the exocyclic amino group of a thymidine nucleotide
<400> 6
   aatgaattga acaaatgagt gagtg 25
<210> 7
   <211> 25
   <212> DNA
   <213> Artificial Sequence
<220>
   <223> internally labelled forward primer (EPL-11-Y-intern4)
<400> 7
   aatgaattga acaaatgagt gagtg 25
<210> 8
   <211> 25
   <212> DNA
   <213> Artificial Sequence
<220>
   <223> internally labelled forward primer (EPL-11-Y-intern5)
<220>
   <221> misc_binding
   <222> (20)..(20)
   <223> ATTO550 linked to the exocyclic amino group of a thymidine nucleotide
<400> 8
   aatgaattga acaaatgagt gagtg 25
<210> 9
   <211> 25
   <212> DNA
   <213> Artificial Sequence
<220>
   <223> internally labelled forward primer (EPL-11-Y-intern6)
<220>
   <221> misc_binding
   <222> (24)..(24)
   <223> ATTO550 linked to the exocyclic amino group of a thymidine nucleotide
<400> 9
   aatgaattga acaaatgagt gagtg 25
<210> 10
   <211> 25
   <212> DNA
   <213> Artificial Sequence
<220>
   <223> unlabelled reverse primer (EPL11/2)
<400> 10
   gaacaactct ggttgtattg tcttc 25